# EUROPÄISCHE PATENTSCHRIFT

(11) **EP 2 361 108 B1**
(45) Veröffentlichungstag und Bekanntmachung des Hinweises auf die Patenterteilung: **21.08.2013**
(21) Anmeldenummer: 09801187.7
(22) Anmeldetag: 08.12.2009
(51) Int. Cl.: A61M 15/00

(54) **AEROSOLTHERAPIEVORRICHTUNG**
AEROSOL THERAPY DEVICE
DISPOSITIF D'AÉROSOLTHÉRAPIE

(30) Priorität: 09.12.2008 DE 102008054431
(43) Veröffentlichungstag der Anmeldung: 31.08.2011
(73) Patentinhaber: PARI Pharma GmbH, 82319 Starnberg (DE)
(72) Erfinder: GALLEM, Thomas, 81371 München (DE); HETZER, Uwe, 81369 München (DE); LÖNNER, Mihaela, 85464 Eicherloh-Finsing (DE)
(74) Vertreter: HOFFMANN EITLE
(86) Internationale Anmeldenummer: PCT/EP2009/066599
(87) Internationale Veröffentlichungsnummer: WO 2010/066714

(56) Entgegenhaltungen:
- WO-A1-00/12161
- WO-A1-2006/078900
- WO-A2-03/022332
- DE-B3-102005 006 374
- DE-C1- 19 953 317
- US-A- 5 584 285
- US-A1- 2001 013 341

## Beschreibung

Die vorliegende Erfindung betrifft eine Aerosoltherapievorrichtung mit den Merkmalen des Oberbegriffs von Anspruch 1.

Eine solche Aerosoltherapievorrichtung ist beispielsweise aus der DE 199 53 317 C2 oder der DE 10 2005 038 619 Al bekannt. Bei derartigen Vorrichtungen werden therapeutisch wirksame oder medikamentenhaltige Flüssigkeiten mittels eines Aerosolgenerators zu einem Aerosol vernebelt, das regelmäßig aus lungengängigen Partikeln besteht. Das Aerosol wird einem Patienten im Rahmen einer Therapie zum Einatmen dargeboten, wodurch die therapeutisch wirksame Flüssigkeit oder das Medikament in die Atemwege des Patienten gelangen. Hierfür wird beim Einatmen des Patienten eine Luftströmung von einem Lufteinlass eines Gehäuses der Aerosoltherapievorrichtung zu einem Luftauslass erzeugt, die den Aerosolgenerator umströmt und das durch den Aerosolgenerator gebildete Aerosol zur Verabreichung mitnimmt. Beim Ausatmen wird die ausgeatmete Luft vorzugsweise möglichst vollständig über ein Rückschlagventil im Bereich des Luftauslasses aus dem Gehäuse ausgegeben, ohne dabei den Prozess der fortgesetzten Aerosolgenerierung bzw. der Mischung des Aerosols zu beeinträchtigen. Je nach Patient können sich die Atemmanöver (Einatmen und Ausatmen) stark unterscheiden. Neben einem durchschnittlichen, gesunden Erwachsenen werden die Aerosoltherapievorrichtungen unter anderem auch von Kindern verwendet. Das Atemmanöver eines gesunden Durchschnittskindes unterscheidet sich jedoch in vielerlei Hinsicht, z.B. der Zahl der Atemzüge pro Minute, den Atemvolumina oder dem Verhältnis zwischen Einatem- und Ausatemdauer. Als weiteres Beispiel eines möglichen Verwenders der Aerosoltherapievorrichtung ist ein Erwachsener Patient mit Emphysem zu nennen, dessen durchschnittliche Zahl der Atemzüge pro Minute zwischen denen eines gesunden Kindes und eines gesunden Erwachsenen liegt. Darüber hinaus unterscheiden sich auch hier die Atemvolumina sowie das Verhältnis zwischen Einatem- und Ausatemdauer voneinander.

Als Folge wird bei Verwendung der Aerosoltherapievorrichtungen durch unterschiedliche Patienten bzw. Anwender ein unterschiedlicher zeitlicher Verlauf des Luftflusses (Strömungsgeschwindigkeit, Durchfluss, etc) durch die Vorrichtung generiert. Insbesondere bei Verabreichung eines Medikaments ist es jedoch von erhöhter Bedeutung eine vorgegebene konstante Dosis verabreichen zu können. Mit anderen Worten ist es erforderlich, dass am Luftauslass der Vorrichtung eine der gewünschten Dosis entsprechende Menge an vernebeltem Medikament bereitgestellt wird. Bei unterschiedlichen Atemmustern durch die Vorrichtung besteht jedoch die Gefahr, dass in Abhängigkeit vom zeitlichen Luftfluss unterschiedliche Mengen am Luftauslass ankommen und damit eine gewünschte Dosiergenauigkeit nicht erreicht werden kann.

Dies ist von besonderer Bedeutung bei Anwendern bei denen sich eine Verschlechterung des Zustands der Lunge und damit eine Veränderung des Inhalationsmanövers von einer zur nächsten Inhalation einstellen. Dies kann bei lungenkranken Patienten zum Beispiel durch Exazerbationen während des Krankheitsverlaufes immer wieder geschehen. Trotzdem oder gerade während diesen Verschlechterungen soll der Anwender (Patient) eine konstante bzw. äquivalente Medikamentendosis appliziert bekommen und dies unabhängig vom Zeitpunkt der Inhalation und des möglichen Inhalationsmanövers.

Darüber hinaus ist aus der WO-A-00/12161 eine Aerosoltherapievorrichtung mit Ultraschallaerosolgenerator bekannt, bei dem stromab des Aerosolgenerators eine Lufteinlassöffnung zur Verwirbelung des Aerosols in einem kegelstumpfförmig gekrümmten Strömungskanal angeordnet ist. Schließlich offenbart die US-A-2001/0013341 einen Düsenvernebler als Aerosoltherapievorrichtung, bei dem ein Ventilgehäuse mit einem Lufteinlassventil im Strömungskanal sitzt und Aerosol an diesem Ventilgehäuse vorbeiströmen muss.

Die Aufgabe der vorliegenden Erfindung beruht nun darin eine Aerosoltherapievorrichtung zu schaffen, die im Wesentlichen unabhängig von dem Anwender (Patienten) sowie dessen Krankheitszustandes, und damit dem erzeugten Atemmuster, am Luftauslass eine im Wesentlichen konstante, d. h. in einem vorgegebenen Bereich liegende Aerosolausgabe ermöglicht.

Diese Aufgabe wird durch die Merkmale des Anspruchs 1 gelöst. Vorteilhafte Weiterbildungen der vorliegenden Erfindung finden sich in den Unteransprüchen.
Der vorliegenden Erfindung liegt der Gedanke zu Grunde den Strömungskanal zwischen dem Aerosolgenerator und dem Luftauslass derart zu gestalten, dass eine möglichst laminare Luft- bzw. Aerosolströmung vom Aerosolgenerator zum Luftauslass erzielbar ist. Durch diese strömungsoptimierte Luftführung können Abscheidungen des Aerosols an den Innenwänden des Gehäuses der Aerosoltherapievorrichtung deutlich reduziert bzw. so gut wie vermieden werden. Hierzu trägt auch bei, dass die Einatemluft den Aerosolgenerator in Form einer "Hüllströmung" umströmt, so dass in unmittelbarer Umgebung des möglichst wenig Turbulenzen entstehen, wodurch eine damit verbundene höhere Abscheidungsrate vermieden werden kann.

Die Medikamentendeposition in der Lunge und damit eine erfolgreiche. Inhalationstherapie hängen im Wesentlichen von der Tröpfchengrößenverteilung und der Ausbringrate des Aerosols sowie dem Atemmuster des Patienten ab. Das Medikamentenaerosol hat unterschiedliche Charakteristika und Wirkungsweisen im Körper. Das Inhalationsgerät beeinflusst die Aerosolgröße (MMD, MMAD), Verteilung (GSD) und Ausbringrate (TOR) des jeweiligen flüssigen Medikamentes. Der Lungenaufbau bzw. -zustand (Morphometrie) des Patienten hat ebenfalls einen deutlichen Einfluss auf die Lungendeposition und den Therapieerfolg.

Dies lässt sich am Besten mit einem Beispiel erklären. Ein wirkungsvolles Medikament gepaart mit einem schlechten Vernebler (Aerosolgenerator) führt zu einem Medikamentenaerosol mit einem größeren MMD. Es werden zu große Aerosoltröpfchen generiert. Diese können den Kehlkopf, Stimmbänder und die ersten Bifurkationen der Atemwege bei der Inhalation nicht passieren. Somit kann zum Beispiel bei einer Kortikoidinhalation in diesen Bereichen vermehrt Medikamentenaerosol abgeschieden werden, was zu Mundpilz (Soor) führen kann. Weiterhin beeinflusst das Atemmanöver die Inhalation. Je nach Verneblersystem kann die Ausbringmenge aus dem Vernebler beeinflusst werden. Zusätzlich wird die Deposition des Medikamentenaerosols in der Lunge durch das Atemmanöver beeinflusst. Hierzu sei auf die veröffentlichte Literatur von W. Bennet "Controlled inhalation of aerosolised therapeutics", Expert Opinion Drug Delivery, 2005, 2(4):763-767, Ashley Pub. und Peter Brand "Total Deposition of therapeutic particles during spontaneous and controlled inhalations", Journal of Pharmaceutical Sciences, Vol . 89, No. 6, June 2000 hingewiesen.

Je nach flüssigem Medikament, Vernbelersystem und daraus resultierenden. Medikamentenaerosol können unterschiedliche Voraussetzungen für die Inhalation geschaffen werden. Weiterhin beeinflusst der Patient mit seinem Atemmanöver und Lungenmorphometrie den Erfolg der Inhalation und können folglich unterschiedliche hohe Nebenwirkungen resultieren.

Die Aerosoltherapievorrichtung der vorliegenden Erfindung umfasst ein Gehäuse. Das Gehäuse kann ein oder mehrteilig aufgebaut sein. Ferner ist in dem Gehäuse ein Aerosolgenerator angeordnet, der dazu dient die eingangs erwähnte Flüssigkeit zu vernebeln. Ferner ist in dem Gehäuse ein Lufteinlass ausgebildet, der stromauf des Aerosolgenerators angeordnet ist sowie ein Luftauslass, der zur Verabreichung des erzeugten Aerosols stromab des Aerosolgenerators ausgebildet ist. Zwischen Lufteinlass und Luftauslass ist ein Strömungsweg gebildet, entlang dem über den Lufteinlass eingebrachte Luft am Aerosolgenerator vorbei zum Luftauslass strömbar ist. In Bezug auf die Terminologie "stromauf" und "stromab" bzw. "Einlass" und "Auslass" wird auf die Strömung bei der Verabreichung des erzeugten Aerosols Bezug genommen, d. h. während eines Einatemzykluses (Inhalation) eines Anwenders. Dabei wird beim Einatmen ein Unterdruck am Luftauslass erzeugt, der eine Luftströmung vom Lufteinlass zum Luftauslass induziert, welche den Aerosolgenerator nach Art einer Hüllströmung umströmt. Die vorliegende Erfindung kennzeichnet sich insbesondere dadurch, dass zwischen dem Aerosolgenerator und dem Luftauslass ein kegelstumpfförmiger Strömungskanal angeordnet ist, dessen Mittelachse gekrümmt verläuft. Der Strömungskanal hat folglich eine in sich verjüngende Hornform mit einem kleineren Öffnungsquerschnitt auf der Seite des Luftauslasses und einem größeren Öffnungsquerschnitt auf der Seite des Aerosolgenerators. Hierbei ist die Mittelachse vorzugsweise kontinuierlich, zum Beilspiel entlang einer Kreisbahn, gekrümmt. Bevorzugt überbrückt der Strömungskanal die Strecke zwischen dem Aerosolgenerator und dem Luftauslass vollständig. Durch den erfindungsgemäßen Aufbau erfolgt eine Strömungsführung mit möglichst wenig Turbulenzen bzw. Verwirbelungen oder Querströmungen. Durch diese StrömungslinienoptimierungStrömungsoptimierung wird erzielt, dass möglichst wenig Aerosol an den Innenwänden des Strömungskanals abgeschieden wird, um das die am Luftauslass abgegebene Aerosolmenge reduziert würde. Es lässt sich durch diese erfindungsgemäße Ausgestaltung damit eine relativ konstante Ausbringungsrate (Total Output Rate, TOR) am Luftauslass und unabhängig vom jeweiligen Atemmanöver und damit des Anwenders realisieren: D. h. bei einem vorgegebenen zu vernebelnden Ausgangsflüssigkeitsvolumen ist ein Output von etwa 70% bis 85% des Ausgangsvolumens unabhängig von dem Anwender realisierbar. Die Outputwerte liegen je nach Ausgestaltung des kegelstumpfförmigen Strömungskanals dabei größer 60% und weisen in der Regel geringe Varianzen unter 15% auf. D. h. bei einem höheren Durchfluss (Strömungsgeschwindigkeit) scheidet sich minimal mehr Aerosol zwischen dem Aerosolgenerator und dem Luftauslass in dem Gehäuse ab, als bei einem geringeren Durchfluss. Damit haben die verbundenen Unterschiede in der Strömungsgeschwindigkeit eine untergeordnete Auswirkung auf die Aerosolabscheidung im Geräteinneren, im Vergleich zu den variablen Inhalation/Exhalation Verhältnissen.

Dabei hat es sich als besonders bevorzugt herausgestellt, dass die Mittelachse mit einem Krümmungsradius in einem Bereich zwischen 40mm und 60mm, vorzugsweise zwischen 45mm und 55mm und am meisten bevorzugt 48mm und 52mm gekrümmt ist. Als eine praktikable Möglichkeit hat sich ein Krümmungsradius vom 50 mm als vorteilhaft erwiesen.

Als Aerosolgenerator kommt vorzugsweise ein Membrangenerator mit einer vibrierbaren Membran, die eine Vielzahl von Öffnungen aufweist zum Einsatz, wobei die Flüssigkeit bei einer Vibration der Membran durch die Öffnungen vernebelt wird. Die Membran ist ein ebenes flächiges Element, das in einer Ebene liegt, die einen Winkel zwischen 65° und 85°, vorzugsweise zwischen 70° und 80° und am meisten bevorzugt zwischen 73° und 77° mit einer Ebene, in der eine Luftaustrittsöffnung (Querschnittsfläche) des Luftauslasses liegt, einschließt. Hierbei hat sich als bevorzugt umsetzbarer Wert ein Winkel von 75° herausgestellt. Dieser Winkel wird vorzugsweise ausschließlich durch den gekrümmten kegelstumpfförmigen Strömungskanal realisiert, wodurch die Ausbreitung des von dem Aerosolgenerator erzeugten Aerosols optimiert werden kann.

Hierbei ist es besonders bevorzugt, wenn die Membran parallel zur Grundfläche des kegelstumpfförmigen Strömungskanals verläuft und sich der Strömungskanal im Wesentlichen unmittelbar an die Membran anschließt. Erfindungsgemäß bildet der Strömungskanal im Bereich seiner Grundfläche die Aerosolmischkammer, in die der Aerosolgenerator vernebelt, so dass die Luftströmung vom Lufteinlass zum Luftauslass das Aerosol auf Ihrem Weg zum Luftauslass mitnimmt.

Des Weiteren ist es während der Einatmung zur gleichmäßigen Beschleunigung und Erzeugung einer Düsenströmung (Vergleichmäßigung der Luftströmung) unabhängig von der Saugkraft vorteilhaft, das Verhältnis von Grundfläche zu Deckfläche des kegelstumpfförmigen Strömungskanals oder von der Grundfläche des kegelstumpfförmigen Strömungskanals zum Querschnitt des Luftauslasses zwischen 1,5 und 3,0, vorzugsweise zwischen 1,8 und 2,5 und am meisten bevorzugt zwischen 1,95 und 2,15 festzulegen. Beispielsweise kann der Strömungskanal an seiner Grundfläche einen Durchmesser von beispielsweise ca. 37mm und an seiner Deckfläche einen Durchmesser von beispielsweise ca. 18mm aufweisen. Alternativ kann auch ein von der Grundfläche zum Luftauslass hin sich verjüngender elliptischer Querschnitt gewählt werden.

Gemäß einer weiteren vorteilhaften Ausführungsform ist zwischen dem Lufteinlass und dem Aerosolgenerator eine Ventileinrichtung angeordnet, die eine Luftströmung nur in Richtung vom Lufteinlass zum Luftauslass gestattet (1-weg bzw. Rückschlagventil), wobei zwischen der Ventileinrichtung und dem Aerosolgenerator, vorzugsweise unmittelbar an den Aerosolgenerator anschließend, eine Expansionskammer (Puffer) gebildet ist. In der Expansionskammer wird die beim Einatmen einströmende Luft unmittelbar vor dem Aerosolgenerator beruhigt, d. h. Turbulenzen werden reduziert bevor der Aerosolgenerator in Form der Hüllströmung umströmt wird. Dieses Merkmal trägt damit weiter zur Vermeidung von Turbulenzen, insbesondere in der nächsten Umgebung des Aerosolgenerators stromab des Aerosolgenerators bei, um auch dadurch Abscheidungen im Gehäuse der Aerosoltherapievorrichtung zu minimieren.

Dabei hat es sich als besonders vorteilhaft erwiesen, wenn das Volumen der Expansionskammer in einem Bereich zwischen 8ml und 18ml, vorzugsweise 10ml und 16ml und am meisten bevorzugt 12ml und 14ml liegt. Dies ist ein gewählter Kompromiss zwischen einer theoretisch großen Expansionskammer die optimal die Luft beruhigt, also die Turbulenzen reduziert und einem kleinen Inhalationsgeräteaufbau, der eine mobile Anwendung erlaubt.

Des Weiteren kann sich an den kegelstumpfförmigen Strömungskanal ein Mundstück mit elliptischem Querschnitt anschließen. Dabei bildet das Mundstück die Schnittstelle zum Anwender. Alternativ kann das Mundstück auch durch eine Atemmaske ersetzt sein. Wenigstens ein Rückschlagventil ist vorzugsweise im Bereich der Verjüngung von dem vorzugsweise kreisrunden Querschnitt der Deckfläche des kegelstumpfförmigen Strömungskanals zu dem elliptischen Querschnitt des Mundstücks in dem Gehäuse vorgesehen. Eine Strömung über das Rückschlagventil in das Gehäuse ist nicht möglich, jedoch eine Luftströmung von dem Luftauslass über das Rückschlagventil aus dem Gehäuse. Dieses Rückschlagventil dient beim Ausatmen dazu die ausgeatmete Luft möglichst unmittelbar aus dem Gehäuse der Aerosoltherapievorrichtung abzuleiten, ohne dass allzu viel frisch erzeugtes Aerosol ausgetragen wird, bzw. die Aerosolproduktion beeinträchtigt wird bzw. Abscheidungsverluste durch Turbulenzen entstehen. Durch die Anordnung des Ventils und Mundstücks in tangentialer Verlängerung des gekrümmten kegelstumpfförmigen Strömungskanals wird vermieden, dass sich im Bereich des Rückschlagventils Strömungsverhältnisse aufbauen, die ein Austragen von Aerosol aus dem kegelstumpfförmigen Strömungskanal durch das Rückschlagventil forcieren. Unter Anderem durch diese spezielle Anordnung wurde es möglich die Aerosolverluste bei kontinuierlicher Aerosolerzeugung und während fortgesetzter Ein- und Ausatmung auf ungefähr 6% des Ausgangsflüssigkeitsvolumens zu reduzieren. Hierzu trägt auch die sich verjüngende strömungsgünstige Ausgestaltung des kegelstumpfförmigen Strömungskanals bei.

Der kegelstumpfförmige Strömungskanal hat gemäß einer bevorzugten Ausführungsform ein Volumen von 30ml-50ml, vorzugsweise 35ml-45ml und am meisten bevorzugt 39ml-42ml. an einem Punkt im Strömungskanal des Geräteinneren (z.B. )

Gemäß einer bevorzugten Ausführungsform wird der Durchfluss an einem Punkt im Strömungskanal des Geräteinneren erfasst und die Steuerung ist derart ausgestaltet, dass sie die Aerosolerzeugung durch den Aerosolgenerator bei Erreichen einer gewünschten maximalen Dosis beendet. Ein Aerosolgenerator mit einer vibrierbaren Membran hat bei vorgegebener Leistung eine konstante Output Rate und die gewünschte Dosis kann über eine Zeitsteuerung und Durchflussmessung bestimmt werden. Hier wäre es beispielsweise denkbar, dass über eine Eingabevorrichtung eine gewünschte zu verabreichende Dosis in die Aerosoltherapievorrichtung eingegeben wird. Die Steuerung errechnet daraufhin einen notwendige Inhalationszeit bzw. Inhalationsvolumen, um die eingegebene Dosis zu erzielen und erfasst kontinuierlich und/oder in vorgegebenen Zeitintervallen den Durchfluss. Ist die errechnete Inhalationszeit für die entsprechende Dosis erzielt, wird die Aerosolerzeugung beendet. Die entsprechende Dosis wurde zu diesem Zeitpunkt verabreicht. Ähnliche Ausgestaltungen sind selbstverständlich auch bei Erfassung von Volumen oder Zeit denkbar.

Weitere Vorteile und Merkmale der vorliegenden Erfindung, die einzeln oder in Kombination mit einem oder mehreren der oben stehenden Merkmale verwirklicht werden können, sind aus der folgenden Beschreibung einer bevorzugten Ausführungsform ersichtlich, welche unter Bezugnahme auf die begleitenden Zeichnungen erfolgt. Hierbei zeigt:
Fig. 1 einen Längsschnitt durch eine erfindungsgemäße Aerosoltherapievorrichtung;
Fig. 2 einen Schnitt durch die in Fig. 1 dargestellte Aerosoltherapievorrichtung entlang der Linie 2-2;
Fig. 3 beispielhaft die Unterschiede im Atemmanöver bei drei unterschiedlichen simulierten Anwendern;
Fig. 4 Messergebnisse bei Verwendung der in den Fig. 1 und 2 dargestellten Aerosoltherapievorrichtung mit unterschiedlichen Atemmanövern und unterschiedlichen Aerosolgeneratoren.

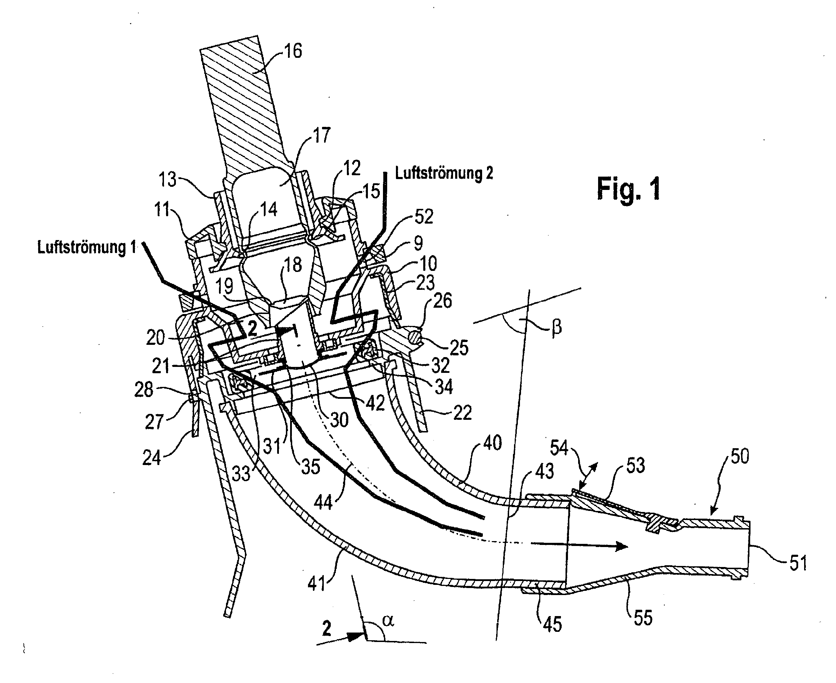

Die in Fig. 1 dargestellte Aerosoltherapievorrichtung umfasst ein mehrteiliges Gehäuse. Das Gehäuse setzt sich aus einem Grundkörper 10 und einem über Gewindegänge mit dem Grundkörper 10 verbundenen relativ zu dem Grundkörper 10 drehbaren Deckel 11 zusammen. Der Deckel 11 weist radial nach innen ragenden Zapfen 12 auf, die in ein Gewinde eines in dem Deckel 11 translatorisch geführten Ampullenhalters greifen. Der Ampulienhalter 18 weist ferner radial nach innen weisende Vorsprünge 14 auf, die in eine Umfangsnut 15 einer Ampulle 16 greifen, so dass die Ampulle lösbar in dem Ampullenhalter gehalten ist. Die Ampulle 16 weist ein Reservoir 17 auf, welches die zu vernebelnde Flüssigkeit sowie ein Luftvolumen enthält. Hierbei kann das Flüssigkeit-zu-Luft-Verhältnis derart eingestellt werden, dass eine zeitlich möglichst gleichmäßige Vernebelung der Flüssigkeit im Reservoir 17 durch den Aerosolgenerator 30 bis zum Ende der Verneblung erzielbar ist. Die Ampulle 16 weist ferner einen Boden 18 mit einer umlaufenden Sollbruchstelle 19 sowie einen sich vom Boden 18 und über diesen hinaus erstreckenden Kragen auf.

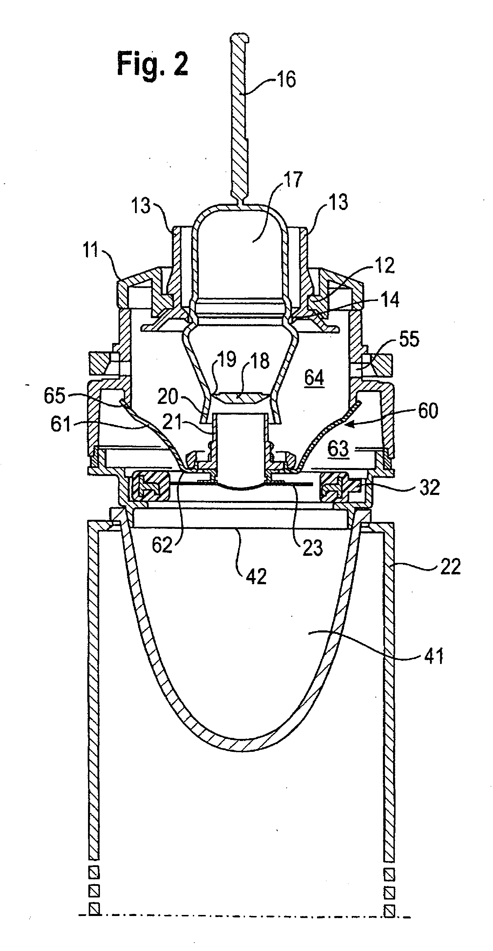

Mit dem Grundkörper 10 des Gehäuses ist ferner ein hohler Aufstechdorn 21 verbunden, um die umlaufende Sollbruchstelle 19 der Ampulle 16 aufzustechen und die im Reservoir 17 enthaltene Substanz dem Aerosolgenerator 30 zur Verfügung zu stellen. Dabei dichtet der umlaufende Kragen gegen den Aufstechdorn ab und übernimmt eine Führungsfunktion beim Öffnen der Ampulle. Die Ampulle ist bei der Darstellung in den Fig. 1 und 2 nicht aufgestochen, d. h. es besteht noch keine Kommunikation zwischen Aerosolgenerator 30 und Reservoir.

Als weiteren Bestandteil weist das Gehäuse einen Ständer 22 auf, der ausgestaltet ist, um die Aerosoltherapievorrichtung auf eine ebene horizontale Fläche, z.B. einen Tisch zu stellen. Dabei ist der Ständer 22 vorzugsweise derart ausgestaltet, dass die Membran 31 des Aerosolgenerators 30 einen Winkel zwischen 30° und 10° zur Horizontalen, vorzugsweise 17,5° zur Horizontalen einschließt. D. h. der Winkel α senkrecht zur Ebene der Membran 31 des Aerosolgenerators 30 zur Horizontalen beträgt ungefähr 107,5°, alternativ beträgt der Nebenwinkel ungefähr 72,5°. Der Ständer 22 ist vorzugsweise über eine Dichtung 23 mit dem Grundkörper 10 verbindbar. Hierzu ist es bevorzugt an dem Grundkörper 10 ein Eingriffselement 25, beispielsweise in Form eines bolzenförmigen Elements, vorzusehen und gleichzeitig an dem Ständer 22 ein Einhängeelement 26 in entsprechender Ausgestaltung zu dem Eingriffselement 25 anzuordnen. Des Weiteren ist angedacht dem Eingriffselement 26 bzw. dem Eingriffselement 25 diametral gegenüberliegend einen Schnapphaken 24 und ein entsprechendes Gegenstück 27 an dem Grundkörper 10 bzw. dem Ständer 22 auszubilden. Durch Einhängen des Einhängeelements 26 in dem Eingriffselement 25 und Verschwenken des Ständers 22, um das Eingriffselement 25 ist es möglich das Eingriffselement 27 (Vorsprung), in dem eine Aussparung 28 aufweisenden Schnapphaken 24 des Grundkörpers 10 zu verrasten und dadurch eine Verbindung des Grundkörpers 10 mit dem Ständer 22 zu erzielen, bei der die beiden Komponenten über die Dichtung gegeneinander gedrückt werden.

Bei dem Aerosolgenerator 30 handelt es sich vorzugsweise um einen Membrangenerator. Er umfasst bei der dargestellten Ausführungsform eine Membran 31, die eine Vielzahl winziger Öffnungen bzw. Löcher im Mikrobereich umfasst, die die Membran vollständig durchdringen. Die Membran 31 ist vorzugsweise über ein piezoelektrisches Element vibrierbar, d. h. sie kann in Schwingung versetzt werden. Durch die Schwingung der Membran wird Flüssigkeit auf einer Seite der Membran, d. h. aus dem Reservoir 17, welche über den hohlen Aufstechdorn 21 einer Seite der Membran 31 zur Verfügung gestellt wird, durch die Öffnungen (nicht sichtbar) treten und auf der anderen Seite der Membran 31 in eine später beschriebene Mischkammer vernebelt werden. Dieses allgemeine Prinzip ist z. B. in der US 5,518,179 erläutert, so dass auf eine detaillierte Beschreibung dieser Funktionsweise hier verzichtet wird.

Erfindungsgemäß wird die Membran 31, bei der es sich um ein flächiges ebenes Element ggf. mit einer mittigen Kuppelausprägung handelt, d. h. sie definiert eine Ebene, über (in den Zeichnungen nicht sichtbare) Stege in einem Rahmen 32 gehalten. Die Membran 31 wie auch der Rahmen 32 sind im Wesentlichen kreisrund bzw. ringförmig gestaltet. Der Rahmen 32 ist gemäß der vorliegenden Ausführungsform mit einem weichelastischen Material ähnlich oder gleich dem Material der Dichtung 23 ummantelt. Als eine Ausführungsform ist diese Ummantelung der Membran 31 durch ein direktes Umspritzen im in Zwei-Komponenten-Kunststoff-Spritzguss-Verfahren realisiert worden. Zwischen der radial innen liegenden Umfangsfläche des die Membran 31 umgebenden Rahmens 32, bestehend aus dem Rahmen und der Ummantelung und der Membran 31 ist mit Ausnahme der Stege entlang des gesamten Membranumfangs ein Zwischenraum 33 gebildet, der einen Durchgang für die später beschriebene Luftströmung bildet, um die genannte Hüllströmung zu generieren. Um den Aerosolgenerator 30 zu montieren, ist in dem Ständer 22 eine Schulter 34 vorgesehen, auf die der Aerosolgenerator 30 bzw. der Rahmen 34 mit der Ummantelung aufgesetzt werden kann. Des Weiteren ist im Bereich des Aufstechdorns 21 dem Aerosolgenerator 30 zugewandt an dem Grundkörper 10 ein Dichtelement 35 mit wenigstens einer umlaufenden flexiblen Dichtlippe vorgesehen. Beim Montieren wird die Membran 31 und zumindest der Bereich der Membran 31, der die winzigen Öffnungen umfasst, von der Dichtlippe des Dichtelements 35 vollständig umgeben, so dass Flüssigkeit aus dem Reservoir 17 über den Dorn 21 dem Membranbereich mit den winzigen Öffnungen zugeführt werden kann, ohne dass Flüssigkeit an der Schnittstelle zwischen der Dichtung 35 und der Membran 31 austreten kann. Bei der Montage wird die Membran 30 über die Schulter 34 und die Verrastung des Ständers 22 mit dem Grundkörper 10 gegen das Dichtelement 35 gedrückt, um eine ausreichende Abdichtung zu erzielen.

Darüber hinaus umfasst das Gehäuse eine Strömungsumlenkung 40, die über eine Art Bajonettverschluss (nicht dargestellt) mit dem Ständer 22 verbindbar ist. Die Strömungsumlenkung 40 bildet einen kegelstumpfförmigen Strömungskanal 41. Dabei erstreckt sich die Grundfläche 42 des kegelstumpfförmigen Strömungskanals 41 im Wesentlichen parallel zur Ebene der Membran 31 und schließt im Wesentlichen direkt an diese an. Die Deckfläche 43 des kegelstumpfförmigen Strömungskanals 41 hingegen verläuft bei der dargestellten Ausführungsform in einem Winkel von 75° zu dieser Ebene oder der Grundfläche 42, wie es durch den Winkel β in Fig. 1 dargestellt ist. Die Grundfläche 42 hat einen kreisrunden Querschnitt mit einem Durchmesser von ungefähr 37 mm, wohingegen die Deckfläche 43 einen kreisrunden Querschnitt mit einem Durchmesser von etwa 18 mm aufweist (Die Maße betreffen die jeweiligen Innendurchmesser). Das Verhältnis der Grundfläche 42 der Deckfläche 43 beträgt damit 2,05. Der kegelstumpfförmige Strömungskanal 41 weist eine Mittelachse 44 auf, die bei der dargestellten Ausführungsform auf einer Kreisbahn verläuft und damit kontinuierlich gekrümmt ist. Bei der dargestellten Ausführungsform beträgt der Krümmungsradius der Mittelachse 50 mm.

An die Deckfläche 43 anschließend bzw. sich von diesem Bereich tangential fortsetzend weist die Strömungsumlenkung 40 eine zylinderförmige Schnittstelle 45 mit kreisrundem Querschnitt entsprechend dem Querschnitt der Deckfläche 43 auf. Diese Schnittstelle 45 dient der Montage eines Mundstücks 50, das ebenfalls Bestandteil des Gehäuses ist. Das Mundstück kann vorzugsweise durch einen reinen Kraftschluss auf der Schnittstelle 45 gehalten werden, so dass es leicht austauschbar und z. B. sterilisierbar ist. Das Mundstück 50 dient bei der dargestellten Ausführungsform ferner dazu den kreisrunden Querschnitt im Bereich der Deckfläche 43 der Strömungsumlenkung 40 auf einen ergonomisch geformten elliptischen Querschnitt am Luftauslass 51 zu verändern. Dabei erfolgt im in Fig. 1 dargestellten Längsschnitt eine Verjüngung 52 in Richtung des Luftauslasses 51. Im Bereich dieser Verjüngung ist ein Rückschlagventil 53 angeordnet, das in Richtung des Pfeils 54 öffnet und schließt. Dieses Rückschlagventil 53 dient beim Ausatmen dazu eine Luftströmung über den Luftauslass 51 durch das Ventil 53 aus dem Gehäuse und damit ein Entweichen der ausgeatmeten Luft aus dem Gehäuse zu ermöglichen. Durch die Anordnung des Ventils und Mundstücks in tangentialer Verlängerung des sich verjüngenden, gekrümmten Strömungskanals entstehen während der Ausatmung Strömungsverhältnisse, die ein Austragen von Aerosol aus dem Bereich zwischen Aerosolgenerator 30 und Rückschlagventil 53 über das Rückschlagventil 53 vermindert. Bei der Exhalation wird die ausgeatmete Luft nicht in die Aerosolmischkammer 41 zurückgeatmet, sondern ohne Störeinflüsse auf das in dem kegelstumpfförmigen Strömungskanal befindliche Aerosol vor der Strömungsumlenkung des Gehäuses 40 direkt nach außen geführt, ohne dabei wesentlich frisch erzeugtes Aerosol mit nach außen zu transportieren. Dieses Aerosol wird stattdessen in dem kegelstumpfförmigen Strömungskanal wie in einer Mischkammer angereichert und steht damit für die nächste Inhalation als angereicherte Aerosolwolke, quasi als Aerosolbolus, zur Verfügung.

Im Grundkörper 10 sind im Bereich der Gewindegänge in die die radial vorragenden Vorsprünge 9 des Deckels 11 greifen mehrere Öffnungen 52 vorgesehen, die als Lufteinlässe fungieren. Diese Lufteinlässe sind auch in Fig. 2 sichtbar. Ferner ist in Fig. 2 eine Ventileinrichtung 60 erkennbar, die aus einer oder mehreren Ventilklappen 61 aufgebaut sein kann. Diese Ventilklappen sind vorzugsweise so ausgestaltet, dass sie um eine Schwenkachse 62 schwenkbar sind und im geschlossenen Ventilzustand dichtend auf einem Ventilsitz 65 aufliegen. Sie sind so angeordnet, dass eine Luftströmung vom Lufteinlass 52 um den Aerosolgenerator 30 zum Luftauslass 41, nicht jedoch in der umgekehrten Richtung möglich ist. Zwischen dem Lufteinlass 52 und der Ventileinrichtung 60 ist ein erster Raum 64 gebildet. Beim Einatmen strömt Luft aus dem Raum 64 über die Ventileinrichtung 60, die sich in diesem Fall öffnet, in einen weiteren Raum 63. Der Raum 63 bildet hierbei eine Expansionskammer, die als Puffer dient und in dem sich die Luftströmung zunächst beruhigt und verlangsamt. Bei der beispielhaften Ausführungsform wird ein Volumen von beispielsweise 13 ml für die Expansionskammer vorgesehen.

Die Ventileinrichtung 60 kann ferner auch derart vorgesehen werden, dass sie in Abhängigkeit von dem am Luftauslass 51 anliegenden Unterdruck beim Einatmen die Lufteinströmung in die Expansionskammer 63 begrenzt. Z. B. kann die Ventilklappe 61 beim Öffnen zunächst an einem ersten Anschlag (nicht dargestellt) anliegen, wodurch ein erster Strömungsquerschnitt zwischen der Ventilklappe 61 und dem Ventilsitz 63 gebildet wird. Wird die Saugkraft erhöht, ist es denkbar, dass die dann flexible Ventilklappe 61 über den ersten Anschlag rutscht und auf einen zweiten Anschlag trifft gegen den die dem Ventilsitz 63 entgegen gesetzte Seite der Ventilklappe 61 abdichtet. In diesem Fall wird ein zweiter Strömungsquerschnitt zwischen dem Anschlag und beispielsweise einer Außenfläche des Grundkörpers 10 gebildet, wobei dieser zweite Strömungsquerschnitt kleiner ist als der vorgenannte erste, so dass der Durchfluss vermindert bzw. ein größerer Strömungswiderstand gebildet wird.

Als weitere Ausführungsform kann die Ventileinrichtung 60 auch als separates Bauteil vorgesehen werden, welches als ein den Atemfluss limitierendes Ventil funktioniert. Dieses Ventil ist an den Einlassöffnungen derart angeordnet, dass ein Unterdruck in dem Verneblungsraum das Ventilelement bewegt um die Einlassöffnungen freizugeben. Die Bewegung des Ventilelementes ist derart beschränkt, dass die Freigabe der Einlassöffnungen nur bis zu einem Schwellenwert des Unterdruckes im Verneblungsraum im Wesentlichen proportional zum Unterdruck ist. Für eine genauere Beschreibung sei auf die DE 101 26 807 und EP 0 895 788 verwiesen.

Im Folgenden wird unter Bezugnahme auf die Fig. 1 und 2 die Funktionsweise der dargestellten und beschriebenen Aerosoltherapievorrichtung erläutert.

Bei der Anwendung nimmt ein Patient das Mundstück 50 in den Mund und beginnt mit einem Atemmanöver. Beim Einatmen wird ein Unterdruck am Luftauslass 51 erzeugt, wodurch sich die Ventilklappe 61 öffnet. Dabei strömt Außenluft über die Einlässe 52 in den Raum 64, durch die Ventilklappen 61 in die Expansionskammer 63, am Aerosolgenerator 30 vorbei über den kegelstumpfförmigen, gekrümmt verlaufenden Strömungskanal 41 zum Luftauslass 51. Währendessen erzeugt der Aerosolgenerator 30 kontinuierlich ein Aerosol, das sich im Bereich der Grundfläche 42 mit der Luft mischt und über den Strömungskanal 41 abgegeben wird. Beginnt der Anwender mit der Ausatmung, wird versucht eine Strömung in umgekehrter Richtung vorzugeben. Durch den dabei entstehenden Überdruck (Staudruck) schließen sich die Einlassventile 61 und verhindern ein Zurückweichen der Aerosolwolke stromaufwärts des Aerosolgenerators in die Expansionskammer 63. Zwischen der Grundfläche 42 und dem Luftauslass 51 kann sich im Bereich der Strömungsumlenkung 40 in dieser Zeit ein neuer Aerosolbolus (verdichtetes Aerosol) aufbauen. Gleichzeitig öffnet das Rückschlagventil 53, um ein Ausströmen der ausgeatmeten Luft aus dem Gehäuse auf möglichst direktem Weg zu gewährleisten, ohne dass ein allzu großer Teil an Aerosol über das Rückschlagventil 53 aus dem Gehäuse der Aerosoltherapievorrichtung austritt. Eine turbulente Durchmischung und damit eine Ausspülung der Aerosolwolke aus dem kegelstumpfförmigen Strömungskanal werden nahezu unterbunden. Im Anschluss folgt wiederum ein Einatemzyklus, wobei der Aersolbolus im gekrümmten Strömungskanal 41 zunächst vollständig ausgetragen wird und erst dann die über die Lufteinlässe 52 und die Ventileinrichtung 60 nachströmende Luft, mit Aerosol kontinuierlich vermischt, inhaliert wird. Dieser Vorgang wiederholt sich bis zum Ende der Therapiesitzung.

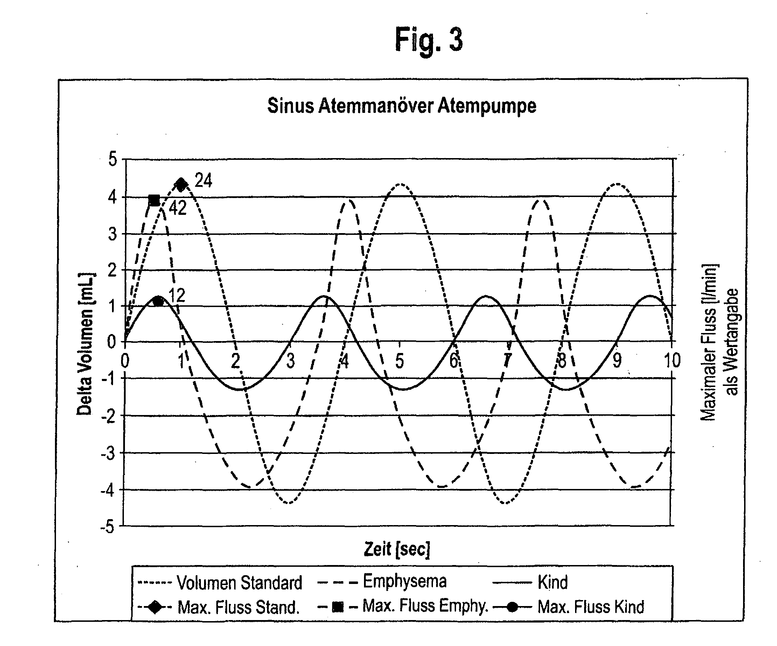

In Versuchen unter Verwendung einer Aerosoltherapievorrichtung, wie sie in den Fig. 1 und 2 dargestellt ist, wurden verschiedene Atemmanöver mittels einer Atempumpe simuliert. Dabei wurde ein Atemmanöver für einen durchschnittlichen Erwachsenen mit einem maximalen Fluss von 24 l/min (Volumen 500ml, Inhalationsverhältnis In:Ex = 50:50, 15 breaths/min), für einen Erwachsenen mit Emphysem mit einem maximalen Fluss von 42 1/min (Volumen 450ml, Inhalationsverhältnis In:Ex = 30:70, 17 breaths/min) und für ein durchschnittliches gesundes Kind von etwa 6 Jahren mit einem maximalen Fluss von 12 l/min (Volumen 150ml, Inhalationsverhältnis In:Ex = 40:60, 20 breaths/min) simuliert (siehe Fig. 3).

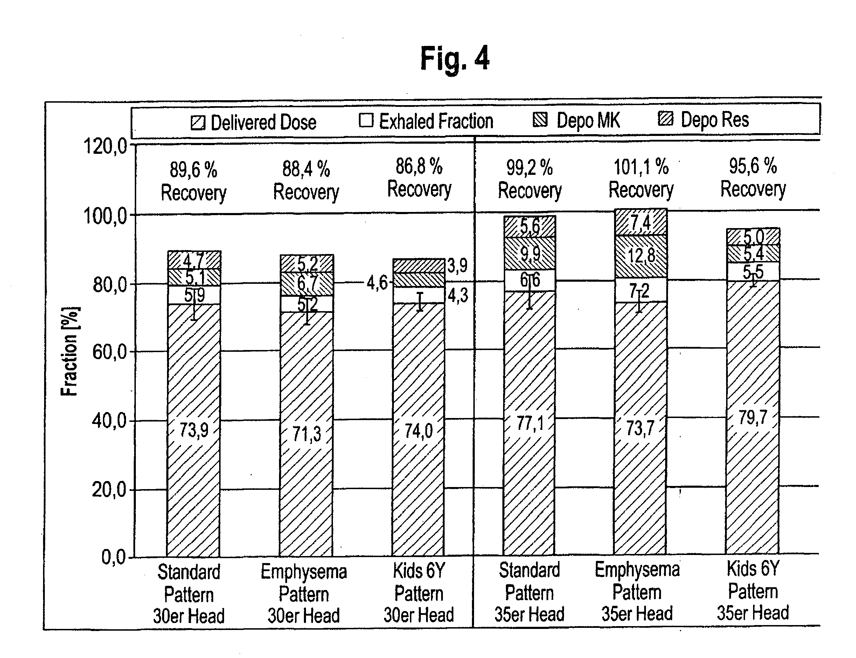

Verwendet wurde eine Ampulle für die verschiedenen Patientenatemmuster mit einem konstanten Füllvolumen (Ausgangsmenge). Am Luftauslass 51 wurde die abgegebene Menge an Aerosol aufgefangen und in Relation zum ursprünglichen Flüssigkeitsinhalt des Reservoirs 17 der Ampulle ermittelt bis die Ampulle vollständig geleert war. Dieser Vorgang wurde für die drei unterschiedlichen Atemmanöver (Fig. 3) wiederholt. Ferner wurde der Bestandteil (Exhaled Fraction) ermittelt, der über das Rückschlagventil 53 abgegeben wurde sowie der im Strömungskanal 41 deponierende Bestandteil (Depo Mk) und der Restbestandteil (Depo Res) im Medikamentenreservoir bzw. in der Ampulle. Der Versuch wurde zusätzlich mit unterschiedlichen Aerosolgeneratoren, die in Fig. 4 mit "30er-Head" und "35er-Head" bezeichnet wurden, die jeweils einen unterschiedlichen medianen Tröpfchendurchmesser (MMD) von 3,22 µm beim "30er-Head" und 3,66 µm beim "35er-Head" haben aufweisen, durchgeführt. Die durchschnittliche Verneblungseffizienz betrug beim "30er-Head" 5,7 mg/J und beim "35er-Head" 7,4 mg/J. Dabei hat sich herausgestellt, dass selbst bei unterschiedlichen Atemmanövern ein relativ konstanter Output am Luftauslass 51 in einem relativ engen Streubereich erzielbar ist. Hier wurden während Atemzugsimulation bei Verwendung des 30er-Heads beispielsweise Aerosolmengen (Delivered Dose) zwischen 71,3 und 74% der Ausgangsmenge gemessen. Gleichzeitig waren die Deposition im Strömungskanal 41 sowie der ausgeatmete Bestandteil (über das Rückschlagventil 43) relativ gering. Auch hat sich herausgestellt, dass durch die erfindungsgemäße Anordnung insbesondere des Strömungskanals 41 sogar bei Verwendung unterschiedlicher Aerosolgeneratoren mit unterschiedlichen Aerosalerzeugungsraten eine relativ konstante Delivered Dose erzielbar ist. Mit anderen Worten ermöglicht es eine Aerosoltherapievorrichtung gemäß der vorliegenden Erfindung auf einfachste Art und Weise, ohne komplizierte elektronische Steuerungen, sowohl unabhängig vom Atemmuster des Anwenders als auch unabhängig von der Spezifikation des verwendeten Aerosolgenerators eine weitgehend konstante Medikamentendosis in Aerosolform zu verabreichen. Eine solche hohe Dosisgenauigkeit ist bei einigen Medikamenten von höchster Bedeutung und war bisher nur bei aufwendigen atemzuggesteuerten Verneblern möglich, nicht jedoch bei Dauerverneblung.

Es versteht sich, dass die vorliegende Erfindung jedoch nicht auf diese explizite Ausgestaltung beschränkt ist und insbesondere nicht auf die beschriebenen Dimensionen eingeschränkt ist. Vielmehr sind Abweichungen im Krümmungsradius, der Neigung der Membran des Aerosolgenerators, des Verhältnisses zwischen Grundfläche und Deckfläche des Strömungskanals sowie der Volumina der Expansionskammer und des Strömungskanals, wie sie oben erläutert wurden, denkbar.

Darüber hinaus ist die vorliegende Aerosoltherapievorrichtung für die Verabreichung der folgenden abschließend aufgelisteten Wirkstoffklassen bzw. -substanzen verwendbar:
Unter den wirksamen Verbindungen sind beispielsweise Substanzen, die aus der Gruppe ausgewählt sind, die aus antientzündlichen Verbindungen, Glucokorticoiden, Beta-agonists, Anticholinergica, Phosphodiesterase-Inhibitoren, Medikamenten gegen Allergien, Antihistaminika, Antioxidantien, Vitaminen, Retinol, Leukotrien-Antagonisten, Mitteln gegen Infektionen, Antibiotika, Antifungiziden, Antivirusmitteln, Mucolytica, Dekongestiva, Antiseptika, Mastzellstabilisatoren, Cytostatika, Immunmodulatoren, Impfstoffen, Mitteln zur Wundheilung, Lokalanästhetika, Plättchenaktivierender Faktor Inhibitoren, Kalium-Kanal-Öffner, Testosteronderivate, Tachikinin- und Kinin-Antagonisten, Interferons, Vasodilatatoren, Vasokonstriktoren, Angiotensin Converting Enzyme (ACE) Inhibitoren, Antidepressiva, Mittel zur Beeinflussung der Signalübermittlung zwischen Zellen, Heparinoide, α-Antitrypsin, Lungensurfactant, Prostaglandine, Endothelin Rezeptor Antagonisten, Vasoactive Intestinal Peptide, Serotonin Rezeptor Antagonisten, Statinen, Calcium Antagonisten, Oligonukleotiden, Peptiden, Proteinen, Phospharimidon, Pflanzenextrakten und Substanzen bezogen aus Pilze besteht.

Beispiele für möglicherweise nützliche anti-entzündliche Verbindungen sind Glukokorticoide wie zum Beispiel Alclomethason, Amcinonid, Betamethason, Beclomethason, Budesanid, Ciclesonid, Clobetasol, Clobetason, Clocortolon, Desonid, Dexamethason, Desoxymethason, Diflorason, Diflucortolon, Fluoconolonacetonid, Flucinonid, Fludroxycortid, Flumetason, Flunisolid, Fluticason, Fluocinonid, Fluocortinbutyl, Fluocortolon, Flupredniden, Halcinonid, Halometason, Hydrocortison, Hydroxycortison, Icomethason, Methylprednisolon, Mometason, Prednicarbat, Prednisolon, Prednison, Rofleponid, und Triamcinolon Acetonid; nicht-steroidale Glukokorticoid Rezeptor Aktivatoren wie zum Beispiel Dehydroepiandrosteron und Derivate wie Dehydroepiandrosteron Sulfat (DHEAS); nicht-steroidale anti-entzündliche Medikamente (NSAIDs) wie zum Beispiel Aceclofenac, Acemetacin, Bromfenac, Diclofenac, Etodolac, Ibuprofen, Indometacin, Nabumeton, Sulindac, Tolmetin, Carprofen, Fenbufen, Fenoprofen, Flurbiprofen, Ketoprofen, Ketorolac, Loxoprofen, Naproxen, Tiaprofensäure, Suprofen, Mefenaminsäure, Meclofenaminsäure, Phenylbutazon, Azaprapazon, Metamizol, Oxyphenbutazon, Sulfinprazon, Lornoxicam, Meloxicam, Piroxicam, Tenoxicam, Celecoxib, Etoricoxib, Lumiracoxib, Parecoxib, Rofecoxib, Valdecoxib, Lodin, Nimesulid, und Licofelon; Prostaglandin Rezeptor Inhibitoren; 5-Lipoxygenase Inhibitoren wie zum Beispiel Zileuton; 5-Lipoxygenase Activating Protein Inhibitoren; Leukotrien Rezeptor Antagonisten wie zum Beispiel Pobilukast, Montelukast, Pranlukast, Roflumilast, und Zafirlukast; Bradykinin Rezeptor Antagonisten; Matrix Metalloproteinase (MMP) Inhibitoren; anti-entzündliche monoklonale Antikörper; und TNF Rezeptor Inhibitoren; einschließlich jeglicher pharmazeutisch annehmbarer Salze, Ester, Isomere, Stereoisomere, Diastereomere, Epimere, Solvate oder andere Hydrate davon, Promedikamente, Derivate oder irgendwelche anderen chemischen oder physikalischen Formen wirksamer Verbindungen, die die entsprechenden wirksamen Reste umfassen.

Die Klasse oder therapeutische Kategorie der Mittel gegen Infektionen, wird hier umfassend verstanden als solche Verbindungen, die gegen bakterielle, virale, und durch Pilze und Protozoen verursachte Infektionen wirksam sind, d. h. solche Verbindungen, die die Klassen der Mikrobizide, Antibiotika, Anti-Virus-Mittel, Fungizide, Anti-Protozoen-Mittel und Antiseptika umfassen.

Beispiele für möglicherweise nützlicher Antibiotika sind
- Penizilline, kombiniert oder nicht kombiniert mit Beta-Lactamase Inhibitoren (wie zum Beispiel Klavulansäure, Sulbactam, und Tazobactam), einschließlich Penizilline mit enges Spektrum wie zum Beispiel Benzylpenizillin, Phenoxymethylpenizillin, Benzathin Benzylpenizillin, Procain benzylpenizillin, Clemizol Benzylpenizillin, Dibenzyletylendiamin Benzylpenizillin; Penizillinase resistente Penizilline mit enges Spektrum wie zum Beispiel Methizillin, Oxazillin, Cloxazillin, Dicloxazillin, Flucloxazillin, Nafzillin, Propizillin, Mecillinam; Beta-Lactamase-resistente Penizilline mit enges Spektrum wie zum Beispiel Temozillin; und Penizilline mit breiterem Spektrum wie zum Beispiel Ampizillin, Amoxizillin, Bacampizillin, Pivampizillin, Ticarzillin, Azlozillin, Piperazillin, Apalzillin, Carbenizillin, Mezlozillin, and Pivmezillinam;
- Cephalosporine, einschließlich Cephalosporine der erste Generation wie zum Beispiel Cefacetril, Cefadroxil, Cefalexin, Cephaloglycin, Cefalonium, Cefaloridin, Cefalotin, Cefapirin, cefatrizin, Cefazaflur, Cefazedon, Cefazolin, Cefradin, Cefroxadin, Ceftezol; Cephalosporine der zweite Generation wie zum Beispiel Cefonicid, Cefprozil, Cefuroxim, Cefuroxim-Axetil, Cefuzonam, Cefaclor, Cefamandol, Ceforanid, Cefotiam, Cefotiam-Hexetil, Loracarbef, Cefbuperazon, Cefmetazol, Cefminox, Cefotetan, Cefoxitin; Cephalosporine der dritte Generation wie zum Beispiel Cefcapen, Cefdaloxim, Cefdinir, Cefditoren, Cefetamet, Cefetamet-Pivoxil , Cefixim, Cefmenoxim, Cefodizim, Cefoperazon, Cefotaxim, Cefpimizol, Cefpodoxim, Cefpodoxim-Proxetil, Cefteram, Ceftibuten, Ceftiofur, Ceftiolen, Ceftizoxim, Ceftriaxon, Ceftazidim, Cefpiramid, Cefsulodin, Latamoxef; Cephalosporine der vierte Generation wie zum Beispiel Cefclidin, Cefepim, Cefluprenam, Cefoselis, Cefozopran, Cefpirom, Cefquinom, Flomoxef; und weitere Cephalosporine wie zum Beispiel Cefaclomezin, Cefaloram, Cefaparol, Cefcanel, Cefedrolor, Cefempidon, Cefetrizol, Cefivitril, Cefmatilen, Cefmepidium, Cefovecin, Cefoxazol, Cefrotil, Cefsumid, Ceftioxid, Cefuracetim, und Ceftobiprol;
- Carbapeneme, einschließlich Imipenem, Imipenem-Cilastatin, Meronenem, Doripenem, Faropenem, Tebipenem, Ertapenem, Panipenem, Biapenem und Ritipenem;
- Monobactams, einschließlich Aztreonam;
- Aminoglycoside, wie beispielsweise Amikacin, Apramycin, Arbekacin, Capreomycin, Gentamycin, Hygromycin B, Isepamycin, Kanamycin, Mupirocin, Neomycin, Netilmicin, Paromomycin, Spectinomycin, Streptomycin, und Tobramycin;
- Macrolide, einschließlich Erythromycin, Azithromycin, Clarithromycin, Dirithromycin, Dithromycin, Roxithromycin, Troleandomycin, Carbomycin A, Josamycin, Kitasamycin, Oleandomycin, Spiramycin, Tylosin, Midecamycin, Rapamycin, Miocamycin, Fluritromycin, Rokitamycin, Rosaramycin, und Telithromycin;
- Gyrase-Inhibitoren oder Fluorochinolones, einschließlich Fluorochinolone der erste Generation, wie zum Beispiel Nalidixinsäure, Oxolinsäure, und Piromidinsäure; Fluorochinolone der zweite Generation, wie zum Beispiel Cinoxacin, Flumequine, Novobiocin, Pipemidinsäure, und Rosoxacin; Fluorochinolone der dritte Generation, wie zum Beispiel Enoxacin, Norfloxacin, Nadifloxacin, Ciprofloxacin, Ofloxacin, Fleroxacin, Lomefloxacin Pefloxacin, Temafloxacin, und Uvofloxacin; und Fluorochinolone der vierte Generation, wie zum Beispiel Balofloxacin, Caderofloxacin, Clinafloxacin, Difloxacin, Garenoxacin, Gatifloxacin, Gemifloxacin, Grepafloxacin, Levofloxacin, Moxifloxacin, Olamufloxacin, Pazufloxacin, Rufloxacin, Sitafloxacin, Sparfloxacin, Tosufloxacin, Trovafloxacin, Ecinofloxacin, und Prulifloxacin;
- Tetracycline, einschließlich Tetracyclin, Chlortetracyclin, Oxytetracyclin, Demeclocyclin, Doxycyclin, Clomocyclin, Lymecyclin, Meclocyclin, Methacyclin, Minocyclin, Penimepicyclin, Rolitetracyclin, Chelocardin, Sancyclin, Apicyclin, Guamecyclin, Meglucyclin, Mepylcyclin, Pipacyclin, Etamocyclin, Penimocyclin, und Tigecyclin;
- Glycopeptide, einschließlich Vancomycin, Teicoplanin, Ristocetin, Avoparcin, Oritavancin, Ramoplanin, Decaplanin, und Peptide 4;
- Polymycine, einschließlich Polymyxin B, Colistin, und Surfactin;
- Lincosamide, einschließlich Lincomycin und Clindamycin;
- Streptogramine, einschließlich Dalfopristin, Quinupristin, Pristinamycin und Virginiamycin;
- Phenicole, einschließlich Chloramphenicol, Tiamphenicol, und Florphenicol;
- Rifamycine, einschließlich Rifampicin, Rifabutin, Rifapentine, und Rifaximin;
- Nicotinsäure Derivate, einschließlich Isoniazid, Ethionamid, Prothionamid, und Pyrazinamid;
- Nitroimidazole, einschließlich Metronidazol, Timidazol, Nimorazol und Ornidazol;
- Nitrofurane, einschließlich Nifurfolin, Nifuroxazid, Nifuroxima, Nifurzid, Nitrofurantoin, und Nitrofurazon;
- Sulfonamide, einschließlich Sulfacarbamide, Sulfamazol, Sulfamazon, Sulfamethizol, Sulfametopirazin, Sulfametoxypiridazin, Sulfametrol, Succinylsulfathiazol, Sulfisoxazol, Sulfamethoxazol, Sulfadiazin, Phtalylsulfacetamid, Phthalylsulfonazol, Phtalylsulfathiazol, Sulfasalazin, Sulfoguanidin, Sulfacetamid, Silber Sulfadiazin, Mafenid acetat, Sulfadoxin, Sulfalen, Cotrimoxazol, Cotrimetrol, Cotrimaxin, und Cotetroxacin;
- andere Antibiotika, einschließlich Plectasin, Dalbavancin, Daptomycin, Ramoplanin, Telavancin, Bacitracin, Tyrothricin, Tygecyclin, Oxazolidinone (wie zum Beispiel Linezolid), Fosfomycin, Cycloserin, Terizidon, Inhibitoren des Dihydropteroat Synthetase, Sulfone, p-Aminosalicylsäure, 2,4-Diaminopirimidines (wie zum Beispiel Bromodiprim, Pyrimethamin, Tetroxyprim), Trimethoprim, Ranbezolid, Ethambutol, Dapson, Fucidinsäure, Terizidon, Ansamycin, Lysostaphin, Iclaprim, Mirocin B17, Clerocidin, Filgrastim, und Pentamidin;

Beispiele potenziell nützlicher Fungizide sind Allylamine und Thiocarbamate, einschließlich Terbinafin, Amorolfin, Naftifin, Butenafin, Tolciclat, und Tolnaftat; Polyene, einschließlich Amphotericin B, Natamycin, Nystatin, Flucocytosin, und Rimocidin; Azole und Triazole, einschließlich Bifonazol, Clotrimazol, Croconazol, Econazol, Fenticonazol, Isoconazol, Miconazol, Oxiconazol, Sertaconazol, Tioconazol, Butoconazol, Sulconazol, Tioconazol, Fluconazol, Itraconazol, Ketoconazol, Voriconazol, Ravuconazol, Posaconazol, Isavuconazol, und Terconazol; Echinocandine, einschließlich Micafungin, Caspofungin, und Anidulafungin; weitere Fungizide, einschließlich Flucytosin, Griseofluvin, Ciclopirox Olamine, Haloprogin, und Undecylensäure; einschließlich jeglicher pharmazeutisch annehmbarer Salze, Ester, Isomere, Stereoisomere, Diastereomere, Epimere, Solvate oder andere Hydrate davon, Promedikamente, Derivate oder irgendwelche anderen chemischen oder physikalischen Formen wirksamer Verbindungen, die die entsprechenden wirksamen Reste umfassen.

Beispiele für möglicherweise nützliche Antivirusmittel sind Amantadine und Derivate, einschließlich Tromantadin und Rimantadin; Neuraminidase Inhibitoren, einschließlich Oseltamivir, Zanamivir, und Peramivir; Nucleoside, einschließlich Acyclovir, Valaciclovir, Penciclovir, Famciclovir, Brivudine, Idoxuridin, Trifluridin, Vidarabin, Ganciclovir, Cidofovir, Entecavir, und Valganciclovir; antiretrovirale Mittel, einschließlich Zidovudin, Abacavir, Adefovir, Didanosin, Lamivudin, Stavudin, Zalcitabin, Delavirdin, Emtricitabine, Efavirenz, Loviride, Nevirapin, Indinavir, Nelfinavir, Ritonavir, Saquinavir, Amprenavir, Lopinavir, Atazanavir, Fosamprenavir, Tipranavir, Darunavir, Adefovir, Enfuvirtid, Lovirid, und Tenofovir; weitere Antivirusmittel, einschließlich Foscarnet, Ribavirin, Arbidol, Docosanol, Edoxudine, Fomivirsen, Fosfonet, Ibacitabin, Imunovir, Imiquimod, Inosin, Interferone, Lysozym, Maraviroc, Moroxydin, Nexavir, Pleconaril, Podophyllotoxin, Vicriviroc, und Viramidin; und feste Antivirusmittel-Kombinationen, einschließlich Atripla, Combivir, Emtricitabin, Trizivir, und Truvada.

Beispiele für mittel wirksam gegen durch Protozoen verursachte Infektionen sind Pentamindin, Cotrimoxazol, Metronidazol, Tinidazol, Nimorazol, und Ornidazol.

Beispiele möglicherweise nützlicher Antiseptika sind Acridin-Derivate, Iod-Povidon, Benzoate, Rivanol, Chlorhexidin, quarternäre Ammonium-Verbindungen, Cetrimide, Biphenylol, Clorofen, und Octenidin.

Beispiele nützlicher Prostaglandine sind Prostacyclin, Epoprostenol, Treprostinil, und Iloprost.

Beispiele für nützliche Endothelin Rezeptor Agonisten sind Bosentran, Sitaxsentan, Ambrisentan, und Darusentan.

Beispiele potentiell nützlicher Phosphodiesterase (PDE) Inhibitoren sind nicht-selektive Methylxantines wie zum Beispiel Theophylline und Pentoxyphylline; und die selektive PDE Isoenzyme Inhibitoren wie zum Beispiel Amrinone, Cilostazol, Benzafentrine, Milrinone, Enoximone, Motapizone, Zardaverine, Tolafentrine, Rolipram, Cilomast, Roflumilast, Sildenafil, Vardenafil, und Tadalafil.

Beispiele für Beta-Agonisten sind die kurz-wirksamen β₂-Sympathicomimetica wie zum Beispiel Salbutamol (Albuterol) Levalbuterol, Terbutaline, Pirbuterol, Procaterol, Metaproterenol, Fenoterol, Bitolterol, und Clenbuterol; und die lang-wirksamen β₂-Sympathicomimetica wie zum Beispiel Salmeterol, Formoterol, Bambuterol, Carmoterol, Arformoterol, Indacaterol, und Picumeterol.

Beispiele potenziell nützlicher Vasokonstriktoren und Dekongestiva, die nützlich sein können, um die Schwellung der Schleimhaut zu reduzieren, sind Alfa-1-sympathicomimetica wie beispielsweise Indanazolin, Naphazolin, Oxymetazolin, Tetryzolin, Tramazolin, Xylometazolin, Phenylephrin, Fenoxazolin, Epinephrin, Ephedrin, Isoprenalin, und Hexoprenalin.

Beispiele für Anticholinergika sind die kurz-wirksamen Anticholinergika wie zum Beispiel Ipratropium, Oxitropium, und Trospium; und die lang-wirksamen Anticholinergika wie zum Beispiel Tiotropium, Revatropate, Glycopyrronium, und Aclidinium.

Beispiele für nützliche Immunomodulatoren sind die oben genannten Glukokorticoide und nicht-steroidale Glukokorticoid Rezeptor Aktivatoren; immunosuppressive monoclonale Antikörper wie zum Beispiel Omalizumab, Infliximab, Adalimumab und Etanercept; Cyclosporin, Tacrolimus, Sirolimus (Rapamycin), Mycophenolat, Dimethylfumarat, Ethylhydrogenfumarat, Methotrexat, Azathioprin, Interferons (alpha, beta, gamma), Tumor Necrosis Faktoren, Cytokine, Interleukine, Echinacea Extract, und Pelargonium Extract.

Beispiele möglicherweise nützlicher Mucolide sind Acetylcystein, Ambroxol, Bromhexin, Carbocystein, Gluthation, Nacystelyn, Dornase alpha, Beifuß, Bromelain, Papain, Clerodendrum, Guaifenesin, Cineol, Guajakol, Myrthol, Mesna, P2Y2-Agonisten (zum Beispiel Denufosol), Heparinoide, Natrium Chloride, Medikamente, die das Eindringen von Chlor- und Natrium betreffen, wie beispielsweise N-(3,5-diamino-6-chlorpyrazin-2-carbony)-N'-{4-[4-(2,3-dihydroxypropoxy)-phenyl]butyl}guanidin-Methanesulfonat (PARION 552-02), Tyloxapol, Lecithin, und rekombinante Lungensurfactant Proteine.

Beispiele für nützliche Antihistaminika sind Diphenhydramin, Carbinoxamin, Doxylamin, Clemastin, Dimenhydrinat, Pheniramin, Chlorphenamin, Dexchlorphenamin, Brompheniramin, Triprolidin, Cyclizin, Chlorcyclizin, Hydroxyzin, Meclizin, Promethazin, Alimemazin, Cyproheptadin, Azatadin, Ketotifen, Azelastine, Levocabastin, Olopatadin, Epinastin, Emedastin, Acrivastin, Astemizol, Cetirizin, Loratadin, Mizolastin, Terfenadin, Fexofenadin, Levocetirizin, und Desloratadin.

Beispiele für Mastzellstabilisatoren sind Cromoglycate, Nedocromil, und Lodoxamid.

Beispiele für nützliche Cytostatika und Metastase-Inhibitoren sind Alkyliermittel, wie Nimustin, Melphanlan, Carmustin, Lomustin, Cyclophosphosphamid, Ifosfamid, Trofosfamid, Chlorambucil, Busulfan, Treosulfan, Prednimustin, Thiotepa, Dacarbazin, und Komplexe von Elementen der Übergangsgruppen (z.B. Ti, Zr, V, Nb, Ta, Mo, W, Pt) wie beispielsweise Carboplatin, Oxiplatin, Cis-Platin und Metallocenverbindungen, wie beispielsweise Titanocendichlorid; Antimetabolite, zum Beispiel Cytarabin, Fluorouracil, Methotrexat, Mercaptopurin, Thioguanin, Hydroxycarbamid, Pemetrexed, und Gemcitabin; Alkaloide, wie Vinblastin, Vincristin, vindesin, und Vinorelbin; Antitumorale Antibiotika, wie beispielsweise Alcarubicin, Bleomycin, Dactinomycin, Daunorubicin, Doxorubicin, Epirubicin, Idarubicin, Mitoxantron, Mitomycin, und Plicamycin; und weitere Cytostatika wie Erlotinib, Gefitinib, Methotrexat, Paclitaxel, Docetaxel, Amsacrin, Estramustin, Etoposid, Beraprost, Procarbazin, Temiposid, Vandetanib, poly-ADP-Ribose-Polymerase (PRAP) Enzym Inhibitoren, Banoxantron, Premetrexed, Bevacizumab, und Ranibizumab.

Beispiele für Verbindungen zur Wundheilung sind Dexpantenol, Allantoin, Vitamine, Hyaluronsäure, alpha-Antitrypsin, anorganische und organische Zinksalze/-verbindungen, und Bismuth- Selen-Salze.

Beispiele für potenziell nützliche Lokalanästhetika schließen Benzocain, Tetracain, Procain, Lidocain und Bupivacain ein.

Beispiele für potenziell nützliche Antioxidanten sind Superoxide Dismutase, Acetylcysteine, Vitamin C, Vitamin E (Tocopherole), Catalase, reduziertes Glutathion, Peroxidase, Harnsäure, β-Caroten, NOX Inhibitoren, Xanthin Oxidase Inhibitoren, Pyruvat- und Gluconatsalze.

Beispiele nützlicher Pflanzenextrakte oder Inhaltsstoffe sind Extrakte aus Kamille, Hamamelis, Echinacea, Calendula, Thymian, Papain, Pelargonium, und Kiefernbäumen; und ätherische Öle wie Myrtol, Pinen, Limonen, Cineol, Thymol, Menthol, Kampfer, Tannin, alpha-Hederin, Bisabolol, Lycopodin, Resveratrol, Vitapherol, und antioxidative Inhaltsstoffe des grünen Tees.

Beispiele möglicher nützlicher Mittel gegen Allergien schließen die oben erwähnte Glukokorticoide, Mastzellstabilisatoren, Anti-Histaminika, Leukotrien Rezeptor Antagonisten, Ziluton, Omalizumab, und Heparinoide ein.

Beispiele für nützliche Angiotensin Converting Enzyme (ACE) Inhibitoren sind Captopril, Lisinopril, Perindopril, Trandolapril, und Cilazapril.

Nützliche Kalium-Kanal-Öffner sind zum Beispiel Cromakalim, Levocromakalim, und Pinacidil.

Beispiel potentiell nützlicher Tachykinin und Kinin Antagonisten sind Nolpitantium, Saredutant, Nepadutant, und Osanetant.

Antisense-Oligonucleotide sind kurze synthetische Stränge aus DNA (oder Analoga), die komplementär oder im Gegensinn zur Zielsequenz (DNA, RNA) sind, welche so entworfen sind, dass sie einen biologischen Vorgang stoppen, wie beispielsweise die Transkription, Translation oder das Splicing. Die dadurch hervorgerufene Inhibierung der Genexpression macht Oligonucleotide, abhängig von ihrer Zusammensetzung, nützlich für die Behandlung vieler Erkrankungen, und zahlreiche Verbindungen werden derzeit klinisch untersucht, wie beispielsweise ALN-RSV01, um das respiratorische Syncytiumvirus zu behandeln, AVE-7279, um Asthma und Allergien zu behandeln, TPI-ASM8, um allergisches Asthma zu behandeln, 1018-ISS, um Krebs zu behandeln.

Beispiele potenziell nützlicher Peptide und Proteine schließen Aminosäure, wie beispielsweise L-Arginin und L-Lysin, Antikörper gegen Toxine, die von Mikroorganismen hergestellt werden, und antimikrobielle Peptide, wie beispielsweise Cecropine, Defensine, Thionine und Cathelicidine ein.

Für jedes dieser und anderer explizit erwähnten Beispiele für Medikamentensubstanzen, die potenziell zur Durchführung der Erfindung nützlich sind, sollen die Verbindungsnamen, die hier angegeben worden sind, so verstanden werden, dass sie auch jegliche pharmazeutisch annehmbaren Salze, Esters, Isomere, Stereoisomere, Diastereomere, Epimere, Solvate oder andere Hydrate, Promedikamente, Derivate oder irgendwelche anderen chemischen oder physikalischen Formen der entsprechenden Verbindungen, die die entsprechenden aktiven Reste umfassen, betreffen.

## Patentansprüche

1. Aerosoltherapievorrichtung umfassend:
ein Gehäuse;
einen Aersolgenerator, der in dem Gehäuse angeordnet ist;
einen Lufteinlass (52), der stromauf des Aerosolgenerators (30) in dem Gehäuse ausgebildet ist; und
einen Luftauslass (51), der stromab des Aerosolgenerators (30) zur Verabreichung des erzeugten Aerosols in dem Gehäuse ausgebildet ist, wobei durch einen Unterdruck am Luftauslass (51) eine Luftströmung vom Lufteinlass (52) zum Luftauslass (51)generierbar ist, die den Aersolgenerator (30) umströmt, **dadurch gekennzeichnet, dass** zwischen dem Aersolgenerator und dem Luftauslass (51) ein kegelstumpfförmiger Strömungskanal (41) angeordnet ist, dessen Mittelachse (44) gekrümmt verläuft, wobei der Strömungskanal (41) im Bereich seiner Grundfläche eine Aerosolmischkammer bildet, in die der Aerosolgenerator vernebelt, so dass eine Luftströmung von Lufteinlass zum Luftauslass das Aerosol auf ihrem Weg zum Luftauslass mitnimmt.

2. Aerosoltherapievorrichtung nach Anspruch 1, bei der die Mittelachse (44) mit einem Krümmungsradius in einem Bereich zwischen 40 und 60 mm, vorzugsweise zwischen 45 und 55 mm, am meisten bevorzugt 48 und 52 mm gekrümmt ist.

3. Aerosoltherapievorrichtung nach Anspruch 1 oder 2, bei der der Aerosolgenerator (30) eine vibrierbare Membran (31) mit einer Vielzahl von Öffnungen (52), durch die eine Substanz vernebelbar ist, aufweist und die Membran (31) in einer Ebene liegt, die einen Winkel zwischen 65°und 85°, vorzugsweise zwischen 70°und 80°, am meisten bevorzugt zwischen 73° und 77° mit einer Ebene in der eine Luftaustrittsöffnung (Querschnittsfläche) des Luftauslasses (51) liegt einschließt.

4. Aerosoltherapievorrichtung nach einem der vorstehenden Ansprüche, bei der der Aerosolgenerator (30) eine vibrierbare Membran (31) mit einer Vielzahl von Öffnungen (52), durch die eine Substanz vernebelbar ist, aufweist und die Membran (31) in einer Ebene liegt, die parallel zur Grundfläche (42) des kegelstumpfförmigen Strömungskanals (41) verläuft.

5. Aerosoltherapievorrichtung nach Anspruch 4, bei der der kegelstumpfförmige Strömungskanal (41) im Wesentlichen unmittelbar an die Membran (31) anschließt.

6. Aerosoltherapievorrichtung nach einem der vorstehenden Ansprüche, bei der das Verhältnis von Grundfläche (42) zur Deckfläche (43) des kegelstumpfförmigen Strömungskanals (41) oder von der Grundfläche (42) des kegelstumpfförmigen Strömungskanals (41) und dem Querschnitt des Luftauslasses (51) zwischen 1,5 und 3,0, vorzugsweise zwischen 1,80 und 2,50 und am meisten bevorzugt zwischen 1,95 und 2,15 liegt.

7. Aerosoltherapievorrichtung nach einem der vorstehenden Ansprüche, bei der zwischen dem Lufteinlass (52) und dem Aerosolgenerator (30) eine Ventileinrichtung (60) angeordnet ist, die eine Luftströmung nur vom Lufteinlass (52) zum Luftauslass (51) gestattet, und bei der zwischen der Ventileinrichtung (60) und dem Aerosolgenerator (30) eine Expansionskammer (63) gebildet ist.

8. Aerosoltherapievorrichtung nach einem der vorstehenden Ansprüche, bei der das Volumen der Expansionskammer (63) in einem Bereich zwischen 8 und 18 ml, vorzugsweise 10 und 16 ml, am meisten bevorzugt 12 und 14 ml liegt.

9. Aerosoltherapievorrichtung nach einem der vorstehenden Ansprüche, bei der sich an den kegelstumpfförmigen Strömungskanal (41) ein Mundstück (50) mit elliptischem Querschnitt anschließt, und wenigstens ein Rückschlagventil (53) im Bereich der Verjüngung (52) von dem kreisrunden Querschnitt der Deckfläche (43) des kegelstumpfförmigen Strömungskanals (41) zu dem elliptischen Querschnitt des Mundstücks (50) in dem Gehäuse angeordnet ist, das nur eine Luftströmung von dem Luftauslass (51) über das Rückschlagventil (53) aus dem Gehäuse gestattet.

10. Aerosoltherapievorrichtung nach einem der vorstehenden Ansprüche, bei der der kegelstumpfförmige Strömungskanal (41) ein Volumen von 30 bis 50ml, vorzugsweise 35 bis 45ml und am meisten bevorzugt 38 bis 42ml aufweist.

11. Aerosoltherapievorrichtung nach einem der vorstehenden Ansprüche, bei der im Bereich des Luftauslasses (51) ein Flusssensor angeordnet ist, der den Luftfluss am Luftauslass (51) erfasst, ferner umfassend eine Steuerung, die ausgestaltet ist, um den Aerosolgenerator (30) basierend auf dem erfassten Luftfluss anzusteuern.

12. Aerosoltherapievorrichtung nach Anspruch 11, bei der der Sensor den Durchfluss am Luftauslass (51) erfasst, und die Steuerung ausgestaltet ist, um die Aerosolerzeugung durch den Aerosolgenerator (30) bei Erreichen eines maximalen Durchflusses zu beenden.

## Claims

1. An aerosol therapy device comprising:
a housing;
an aerosol generator arranged in the housing;
an air inlet (52) formed in the housing upstream of the aerosol generator (30); and
an air outlet (51) formed in the housing downstream of the aerosol generator (30) to administer the generated aerosol, with a flow of air from the air inlet (52) to the air outlet (51), which passes around the aerosol generator (30), being generable owing to a negative pressure at the air outlet (51), **characterised in that** a frustoconical flow channel (41) is disposed between the aerosol generator and the air outlet (51), the central axis (44) of which extends in a curved manner, wherein the flow channel (41) forms an aerosol mixing chamber, into which the aerosol generator nebulises, in the region of its base so that an air flow from the air inlet to the air outlet takes the aerosol with it on its path to the air outlet.

2. An aerosol therapy device according to claim 1, wherein the central axis (44) is curved with a radius of curvature in the range of between 40 and 60 mm, preferably between 45 and 55 mm, most preferred between 48 and 52 mm.

3. An aerosol therapy device according to claim 1 or 2, wherein the aerosol generator (30) comprises a vibratable membrane (31) having a plurality of openings (52) through which a substance can be nebulised, and the membrane (31) lies in a plane that forms an angle of between 65° and 85°, preferably between 70° and 80°, most preferred between 73° and 77°, with a plane in which an air outlet opening (cross-sectional area) of the air outlet (51) lies.

4. An aerosol therapy device according to one of the preceding claims, wherein the aerosol generator (30) comprises a vibratable membrane (31) having a plurality of openings (52) through which a substance can be nebulised, and the membrane (31) lies in a plane that extends parallel to the base surface (42) of the frustoconical flow channel (41).

5. An aerosol therapy device according to claim 4, wherein the frustoconical flow channel (41) substantially directly adjoins the membrane (31).

6. An aerosol therapy device according to one of the preceding claims, wherein the ratio of the base surface (42) to the upper surface (43) of the frustoconical flow channel (41) or of the base surface (42) of the frustoconical flow channel (41) to the cross-section of the air outlet (51) is between 1.5 and 3.0, preferably between 1.80 and 2.50 and most preferred between 1.95 and 2.15.

7. An aerosol therapy device according to one of the preceding claims, wherein a valve arrangement (60) is disposed between the air inlet (52) and the aerosol generator (30), which only allows a flow of air from the air inlet (52) towards the air outlet (51), and wherein an expansion chamber (63) is formed between the valve arrangement (60) and the aerosol generator (30).

8. An aerosol therapy device according to one of the preceding claims, wherein the volume of the expansion chamber (63) lies in a range of between 8 and 18 ml, preferably 10 and 16 ml, most preferred between 12 and 14 ml.

9. An aerosol therapy device according to one of the preceding claims, wherein a mouthpiece (50) having an elliptical cross-section attaches to the frustoconical flow channel (41), and at least one non-return valve (53) is arranged in the housing in the region of the tapering (52) from the circular cross-section of the upper surface (43) of the frustoconical flow channel (41) to the elliptical cross-section of the mouthpiece (50), said valve only allowing a flow of air out of the housing from the air outlet (51) via the non-return valve (53).

10. An aerosol therapy device according to one of the preceding claims, wherein the frustoconical flow channel (41) has a volume of 30 to 50 ml, preferably 35 to 45 ml and most preferred 38 to 42 ml.

11. An aerosol therapy device according to one of the preceding claims, wherein a flow sensor is arranged in the region of the air outlet (51), which detects the airflow at the air outlet (51), further comprising a control that is designed to control the aerosol generator (30) based on the detected airflow.

12. An aerosol therapy device according to claim 11, wherein the sensor detects the flow rate at the air outlet (51) and the control is designed such that it terminates aerosol generation by the aerosol generator (30) upon reaching a maximum flow rate.

## Revendications

1. Dispositif d'aérosolthérapie, comprenant :
un boîtier ;
un générateur d'aérosol, disposé dans le boîtier ;
une entrée d'air (52) réalisée dans le boîtier en amont du générateur d'aérosol (30) ; et
une sortie d'air (51), réalisée dans le boîtier en aval du générateur d'aérosol (30) pour l'administration de l'aérosol généré, un flux d'air de l'entrée d'air (52) vers la sortie d'air (51) pouvant être généré par une dépression au niveau de la sortie d'air (51), ledit flux d'air enveloppant le générateur d'aérosol (30), **caractérisé en ce qu'**un canal d'écoulement tronconique (41) s'étend entre le générateur d'aérosol et la sortie d'air (51), dont l'axe médian (44) est incurvé, ledit canal d'écoulement (41) formant un compartiment de mélange d'aérosol au niveau de sa surface de base, que nébulise le générateur d'aérosol, si bien qu'un flux d'air circulant de l'entrée d'air à la sortie d'air entraîne l'aérosol sur son chemin vers la sortie d'air.

2. Dispositif d'aérosolthérapie selon la revendication 1, où l'axe médian (44) est incurvé suivant un rayon de courbe compris entre 40 et 60 mm, préférentiellement entre 45 et 55 mm, et tout particulièrement entre 48 et 52 mm.

3. Dispositif d'aérosolthérapie selon la revendication 1 ou 2, où le générateur d'aérosol (30) comporte une membrane (31) vibrante avec une pluralité d'ouvertures (52) par lesquelles une substance est nébulisable, et où la membrane (31) est située sur un plan formant un angle compris entre 65° et 85°, préférentiellement entre 70°et 80°, et tout particulièrement entre 73° et 77° avec un plan où se trouve une ouverture d'échappement d'air (surface de section transversale) de la sortie d'air (51).

4. Dispositif d'aérosolthérapie selon l'une des revendications précédentes, où le générateur d'aérosol (30) comporte une membrane (31) vibrante avec une pluralité d'ouvertures (52) par lesquelles une substance est nébulisable, et où la membrane (31) est située sur un plan qui s'étend parallèlement à la surface de base (42) du canal d'écoulement tronconique (41).

5. Dispositif d'aérosolthérapie selon la revendication 4, où le canal d'écoulement tronconique (41) est sensiblement immédiatement adjacent à la membrane (31).

6. Dispositif d'aérosolthérapie selon l'une des revendications précédentes, où le rapport entre la surface de base (42) et la surface de couverture (43) du canal d'écoulement tronconique (41) ou entre la surface de base (42) du canal d'écoulement tronconique (41) et la section transversale de la sortie d'air (51) est compris entre 1,5 et 3,0, préférentiellement entre 1,80 et 2,50 et tout particulièrement entre 1,95 et 2,15.

7. Dispositif d'aérosolthérapie selon l'une des revendications précédentes, où un dispositif de valve (60) est situé entre l'entrée d'air (52) et le générateur d'aérosol (30), lequel ne permet un flux d'air que de l'entrée d'air (52) vers la sortie d'air (51), et où un compartiment d'expansion (63) est formé entre le dispositif de valve (60) et le générateur d'aérosol (30).

8. Dispositif d'aérosolthérapie selon l'une des revendications précédentes, où le volume du compartiment d'expansion (63) est compris entre 8 et 18 ml, préférentiellement entre 10 et 16 ml, et tout particulièrement entre 12 et 14 ml.

9. Dispositif d'aérosolthérapie selon l'une des revendications précédentes, où un embout (50) de section transversale elliptique est adjacent au canal d'écoulement tronconique (41), et où au moins une valve anti-retour (53) est disposée dans le boîtier au niveau du rétrécissement (52) de la section transversale circulaire de la surface de couverture (43) du canal d'écoulement tronconique (41) à la section transversale elliptique de l'embout (50), laquelle ne permet un flux d'air hors du boîtier que par l'intermédiaire de la valve anti-retour (53) depuis la sortie d'air (51).

10. Dispositif d'aérosolthérapie selon l'une des revendications précédentes, où le canal d'écoulement tronconique (41) a un volume compris entre 30 et 50 ml, préférentiellement entre 35 et 45 ml et tout particulièrement entre 38 et 42 ml.

11. Dispositif d'aérosolthérapie selon l'une des revendications précédentes, où un capteur de flux est disposé au niveau de la sortie d'air (51), lequel détecte le flux d'air à la sortie d'air (51), et comprend en outre une commande prévue pour commander le générateur d'aérosol (30) sur la base du flux d'air détecté.

12. Dispositif d'aérosolthérapie selon la revendication 11, où le capteur détecte le flux d'air à la sortie d'air (51), et où la commande est prévue pour arrêter la génération d'aérosol par le générateur d'aérosol (30) quand un débit maximal est atteint.
